# EUROPÄISCHE PATENTSCHRIFT

(11) **EP 3 134 435 B1**
(45) Veröffentlichungstag und Bekanntmachung des Hinweises auf die Patenterteilung: **27.06.2018**
(21) Anmeldenummer: 14727392.4
(22) Anmeldetag: 20.04.2014
(51) Int. Cl.: C07K 14/71, C07K 16/00, C12N 5/00, G01N 33/50, C12N 15/63, G01N 33/531, G01N 33/569, C12N 5/16

(54) **BIOMOLEKÜLE FREISETZENDE ZELLE UND DEREN SELEKTION MITTELS EINES OBERFLÄCHENPROTEINS**
BIOMOLECULE-RELEASING CELL AND SELECTION THEREOF BY MEANS OF A SURFACE PROTEIN
CELLULE LIBÉRANT DES BIOMOLÉCULES ET SA SÉLECTION AU MOYEN D'UNE PROTÉINE DE SURFACE

(43) Veröffentlichungstag der Anmeldung: 01.03.2017
(73) Patentinhaber: New/era/mabs GmbH, 14482 Potsdam (DE)
(72) Erfinder: LISTEK, Martin, 13059 Berlin (DE); HANACK, Katja, 14169 Berlin (DE); MICHEEL, Burkhard, 17268 Gerswalde (DE)
(74) Vertreter: Simandi, Claus
(86) Internationale Anmeldenummer: PCT/DE2014/000205
(87) Internationale Veröffentlichungsnummer: WO 2015/161835

(56) Entgegenhaltungen:
- EP-A1- 2 272 860
- EP-B1- 0 667 896
- EP-B1- 1 141 271
- WO-A1-2009/010474
- WO-A1-2012/085911
- WO-A1-2012/164320
- WO-A2-03/012449
- US-A1- 2006 073 095
- LISTEK M ET AL: "Insertion of artificial cell surface receptors for antigen-specific labelling of hybridoma cells", IMMUNOLOGY, Bd. 137, Nr. Suppl. 1, P1502, September 2012 (2012-09), Seite 185, 651, XP002734086,
- BECKETT D ET AL: "A minimal peptide substrate in biotin holoenzyme synthetase-catalyzed biotinylation", PROTEIN SCIENCE, WILEY, US, Bd. 8, Nr. 4, 1. Januar 1999 (1999-01-01), Seiten 921-929, XP002971557, ISSN: 0961-8368

## Beschreibung

Die vorliegende Erfindung betrifft ein Verfahren zur Selektion von Zellen, die Biomoleküle, insbesondere Proteine, beispielsweise monoklonale Antikörper, freisetzen sowie hierfür verwendbare Mittel.

Bereits früher gab es Bestrebungen, Antikörper produzierende Hybridomzellen zu selektieren, bei denen die von der produzierenden Zelle sezernierten Antikörper an deren Zelloberfläche gebunden werden sollten, um die so oberflächenmarkierten Zellen mittels fluoreszenzmarkierter Nachweis-Antikörper zu identifizieren, die identifizierten Zellen zu isolieren und dann zwecks Gewinnung der von einer Zelle produzierten Antikörper selektiv zu vermehren.

EP 1 141 271 B1 offenbart ein Verfahren zur Selektion von monoklonalen Antikörpern, das die Fusion von B-Lymphozyten mit Myelomzellen zu antikörperproduzierenden Hybridomzellen umfasst, wobei die Antikörper auf der Zelloberfläche der Hybridomzellen mittels eines Antikörper-Bindeproteins präsentiert werden, sowie die Bindung der Antikörper an Antigene. Das Antikörper-Bindeprotein wird dabei über die Myelomzelle oder über Expressionsvektoren in die Hybridomzelle eingeführt. Das Antikörper-Bindeprotein weist eine von der Spezifität der variablen Regionen des Antikörpers unabhängige Antikörper-Bindestelle und einen Membrananker auf. Der produzierte Antikörper wird über diese Bindestelle so an der Zelloberfläche exponiert, dass seine spezifischen Bindungsregionen mit Epitopen, gegen die der produzierte Antikörper gerichtet ist, interagieren kann.

Bei diesem Verfahren gemäß dem Stand der Technik ist die Markierung der Zelloberflächen abhängig von der Affinität des Antikörper-Bindeproteins zu dem freigesetzten Antikörper. So ist es möglich, dass Zellen, die in maximaler Menge den gewünschten, gegenüber dem Antigenepitop hochspezifischen Antikörper sezernieren, im Rahmen der Selektion entgegen der Zielsetzung nicht als optimal erkannt werden, da ihre Affinität zu dem Antikörperbindeprotein kleiner als die eines gegenüber dem Antigenepitop weniger affinen, suboptimalen Antikörpers ist, wobei die geringere Affinität des optimalen Antikörpers zu dem Antikörper-Bindeprotein im Vergleich zu dem suboptimalen Antikörper selbst durch größere Produktionsmengen des optimalen Antikörpers gegebenenfalls nicht kompensiert werden kann. Dieser nachteilige Effekt erklärt sich durch kompetitive Verdrängung des möglicherweise optimalen Antikörpers durch Bindeprotein-affinere suboptimale Antikörper. Zudem kann die Bindung eines sezernierten Antikörpers an das Antikörper-Bindeprotein negativen Einfluss auf die Expression und Sezernierung des gewünschten Antikörpers haben. Weiterhin nachteilig ist die Beschränkung des Selektionsverfahrens auf Antikörper aufgrund des Antikörper-Bindeproteins. Andersartige künstliche oder natürliche Biomoleküle werden von diesem Verfahren nicht erfasst.

EP 0 667 896 B1 offenbart ein Verfahren mit gleicher Zielsetzung wie EP 1 141 271 B1, zum Selektieren oder Analysieren von Zellen entsprechend einem Produkt, das durch die Zellen sekretiert und freigesetzt wird. Neben vergleichbaren Varianten wird dabei die Zelloberfläche chemisch mittels Sulfosuccinimidyl-6-(biotinamido)-hexanoat behandelt, so dass die Zelloberfläche nach dieser Behandlung unspezifisch kovalent gebundenes Biotin aufweist. An dieses Biotin wird als Einfang-Komponente ein zuvor mit Avidin konjugierter Antikörper spezifisch über den Avidin-Anteil gebunden, wobei die Bindung zwischen Avidin und Antikörper über die zu Thiolgruppen reduzierten Disulfidbindungen des Antikörpermoleküls und eine in das Avidin eingefügte reaktive Maleimidgurppe erfolgt unter Erhalt der gegen beide Isotypen der leichten Ketten eines produzierten Antikörpers gerichteten Antigenbindungsstellen. Ähnlich wie in EP 1 141 271 B1 offenbart, umfasst die Einfangkomponente einen Antikörper oder Antikörperfragmente, deren gegen das von der Zelle freigesetzte Produkt gerichtete Bindungsstellen an der Zelloberfläche präsentiert werden. Nachteilig ist hier jedoch die mangelnde Produktspezifität der Bindungsstellen, die lediglich gegen die allgemein in Antikörpermolekülen enthaltenen leichten Ketten gerichtet sind. Darüber hinaus ist es nachteilig, dass dieses Verfahren den erwähnten Schritt einer chemischen Biotinylierung umfasst, der eine Abtrennung der zu behandelnden Zellen aus ihrem Wachstumsmedium mit anschließender Resuspendierung in biotinylierungsreagenzhaltiger Lösung erforderlich macht. Insbesondere bei der Produktion von monoklonalen Antikörpern verwendete, frisch aus Myelom- und B-Zellen fusionierte Hybridomzellen können durch diesen Schritt unerwünschten Schaden nehmen. Weiterhin erfordert die chemische Biotinylierung eine jeweils zellspezifische Titration der zu verwendenden Menge an Biotinylierungsreagenz. Da das Reagenz unspezifische Bindungen von Biotin an Oberflächenproteine bewirkt, kann nicht ausgeschlossen werden, dass durch die Modifikation der Oberflächenproteine vermittelte Signalkaskaden im Zellstoffwechsel initiiert werden, die zu dem Nachteil einer verminderten Expression der gewünschten Zellprodukte führen, was beispielsweise die Ausbeute an erwünschten monoklonalen Antikörpern empfindlich verringern kann.

Sowohl EP 1 141 271 B1 als auch EP 0 667 896 B1 offenbaren Verfahren, die keine verlässliche Identifizierung und Selektion des optimalen Antikörpers erlauben. Denn gemäß EP 1 141 271 B1 ist die Affinität des sekretierten Antikörpers zu dem membranständigen Antikörper-Bindeprotein unabhängig von der Affinität des sekretierten Antikörpers zu dem Epitop, gegen das seine spezifischen Bindungsstellen gerichtet sind. Die Affinität des Antikörpers zu einem Zielepitop ist aber das Hauptziel der Selektion. Wird der Identifizierung ein nicht hinreichend kontrollierbarer und quantifizierbarer Schritt vorangestellt, so wirkt sich dies negativ auf die Genauigkeit des gesamten Selektionsvorgangs aus. Im Falle des Verfahrens gemäß EP 0 667 896 B1 ist der Biotinylierungsschritt zwar kontrollierbar und letztlich auch quantifizierbar, jedoch nur mit dem Aufwand einer zellspezifischen Titration zur Bestimmung der einzusetzenden Menge an Reagenz und einer chemischen Behandlung, die Verfahrensschritte erforderlich macht, die die Vitalität der Zellen und ihre Fähigkeit zur Expression des zu produzierenden Proteins erheblich beeinträchtigen oder zunichte machen können. Erschwerend kommt hinzu, dass das oberflächenexponierte Biotin einen avidinkonjugierten Fangantikörper bindet, der lediglich gegen die leichten Ketten des produzierten Antikörpers gerichtet ist, also ebenfalls nicht die das Hauptziel der Selektion darstellende Affinität der variablen Antikörperregionen zu einem bestimmten Epitop des zur Immunisierung verwendeten Antigens zum Gegenstand hat sondern eine allenfalls bezüglich der leichten Ketten isotypenspezifische Affinität, die die Identifizierung des optimal an ein Zielepitop bindenden unter den von einem Zellgemisch produzierten Antikörpern nicht erlaubt. Um zu verhindern, dass suboptimale Produkte, die von möglichst nicht zu selektierenden Zellen freigesetzt werden, an die Einfangkomponente der das optimale Produkt freisetzenden Zelle binden, wird gemäß EP 0 667 896 B1 ein spezielles Medium verwendet, dass die Diffusion freigesetzer Produkte aus der unmittelbaren Umgebung der sie produzierenden Zelle hinaus aufgrund niedriger Permeabilität hemmen soll. Diese Vorgehensweise ist nachteilig, da sie einen Wechsel des Mediums erforderlich macht, wobei sowohl der Wechsel als auch das niedrig-permeable Medium selbst negative Effekte auf Zellvitalität, Produktion und Freisetzung der Produkte haben kann. Ein weiterer Nachteil, der in der chemischen Biotinylierung liegt, ist die Möglichkeit einer unerwünscht hohen Dichte an auf der Zelloberfläche exponiertem Biotin, die aufgrund der Tatsache, dass jedes Avidintetramer vier Biotin-Bindungsstellen aufweist, zu einer unerwünschten Vernetzung der exponierten Biotinmoleküle führen kann, die die Überlebensfähigkeit insbesondere frisch durch Zellfusion erschaffener Hybridomzellen senken kann Listek M et al., Immunology, Bd. 137 (Suppl. 1), P1502, September 2012, beschreiben ein Verfahren zur Selektion von Hybridomzellen mit einem Oberflächenrezeptor aus einer Vielzahl von Hybridomzellen, die eine Konjugation eines spezifischen Antigens an den Zelloberflächenrezeptor umfasst.

Es stellt sich daher die Aufgabe, die oben benannten Nachteile des Stands der Technik weistestgehend zu überwinden und die Mittel für ein verlässliches und zielführendes Selektionsverfahren für Biomoleküle freisetzende Zellen anhand der spezifischen Bindungseigenschaften der freigesetzten Biomoleküle bereit zu stellen, um ein optimiertes Verfahren zu ermöglichen, das die Bindung der freigesetzten Biomoleküle an die Oberfläche der sie produzierenden Zellen umfasst.

Die Aufgabe wird durch ein Verfahren gemäß Anspruch 1, ein Oberflächenprotein gemäß Anspruch 10 gelöst, wobei sich bevorzugte Ausführungsvarianten aus den jeweiligen Unteransprüchen ergeben.

Ausgangspunkt für die Erfindung sind Biomoleküle, insbesondere Proteine, wobei das Biomolekül von der Zelle produziert und sezerniert, im Regelfall also in das umgebende Medium abgegeben wird. Bei den sezernierten Biomolekülen kann es sich um jede Art von Biomolekülen handeln, die von einer Zelle produziert und im Falle von Proteinen regelmäßig unter Abspaltung eines Signalpeptids, sezerniert werden, aber auch um Sekundärstoffe, wie beispielsweise Hormone, Antibiotika oder Alkaloide. Die sezernierten Biomoleküle können beispielsweise auch mittels gentechnischer Verfahren erschaffene Proteinkonstrukte, Antikörper oder Antikörperfragmente oder natürlicherweise von Zellen sezernierte Proteine sein.

Für das angestrebte optimierte Selektionsverfahren wird die Biomoleküle freisetzende Zelle mit einem Oberflächenprotein ausgestattet, das eine extrazellulär exponierte Ligationspeptidsequenz zur enzymatischen Konjugation eines Adapterliganden aufweist, der geeignet ist für die mittelbare oder unmittelbare Kopplung einer wenigstens eine spezifische Bindungsstelle für die freigesetzten Biomoleküle aufweisenden molekulare Fängerstruktur abseits der spezifischen Bindungsstelle. Die Ligationspeptidsequenz ist dabei nicht mit dem aus dem Stand der Technik bekannten Antikörper-Bindeprotein zu verwechseln. Denn anstatt einen Antikörper lediglich über seinen Fc-Teil zu binden, dient die Ligationspeptidsequenz der Bindung eines Adapterliganden, der das Andocken einer nahezu beliebigen molekularen Fängerstruktur ermöglicht. Bevorzugt kommt hierbei Biotin zum Einsatz, das aus Vollmedium unter schonenden Bedingungen mittels einer aus E. coli gewonnenen BirA-Ligase mit der Ligationspeptidsequenz im Verhältnis 1:1 konjugiert wird. Allerdings ist das Verfahren deshalb nicht auf diese Vorgehensweise beschränkt. Auch andere geeignete Ligationspeptidsequenzen und von Biotin verschiedene Adapterliganden können Verwendung finden. Es ist auch möglich, auf die getrennte Anbindung es Adapterliganden und der Fängerstruktur zu verzichten und beispielsweise ein Konstrukt enzymatisch mit der Ligationspeptidsequenz zu konjugieren, das bereits die gewünschten Eigenschaften des Fängerproteins aufweist und ein entsprechendes Epitop präsentiert.

Das Oberflächenprotein ist vorzugsweise mittels eines membranständigen Anteils in der Zellmembran verankert. Alternativ kann es aber auch mehrere transmembrane Domänen aufweisen oder auf andere Art mit der Zelloberfläche verbunden sein, beispielsweise über Interaktionen mit ihrerseits bereits membranständigen nativen Proteinen.

Wegen der wirtschaftlichen Bedeutung monoklonaler Antikörper ist es von Vorteil, das Verfahren und die dafür bereit zu stellenden Mittel auf Hybridomzellen abzustimmen, die monoklonale Antikörper produzieren und freisetzen. Diese Zellen werden deshalb bevorzugt mit dem Oberflächenprotein ausgestattet, wobei die zu verwendende Fängerstruktur eine antigene Molekularstruktur ist und die spezifische Bindungsstelle, die diese antigene Molekularstruktur aufweist, ein Epitop, das von den freigesetzten Antikörpern spezifisch über ihre variablen Regionen gebunden wird. Es können auch solche Zellen verwendet werden, die lediglich Antikörperfragmente freisetzen.

In den seltensten Fällen wird es gelingen, ein natives Oberflächenprotein mit den für das Verfahren erforderlichen Eigenschaften in geeigneten Zellen aufzufinden. Vorzugsweise wird daher eine Protein freisetzende Zelle künstlich mit aus dem Stand der Technik bekannten Methoden dazu veranlasst, ein solches, aus verschiedenen funktionellen Domänen zusammengesetztes Oberflächenprotein zu exprimieren oder eine Vorstufe, die für die gewünschte Lokalisierung an der Zelloberfläche, regelmäßig im Rahmen des Zellstoffwechsels, modifiziert wird.

Es hat sich für die stabile Expression eines für das Oberflächenprotein oder dessen translozier- und prozessierbare Vorstufe codierenden Vektors als vorteilhaft erwiesen, vor der Fusionierung zur Hybridomzelle die hierfür verwendete Myelomzelle mit den Erbinformationen für das Oberflächenprotein zu transfizieren.

Für die Verwendbarkeit der bereit zu stellenden Zellen in dem angestrebten optimierten Selektionsverfahren ist es förderlich, dass die Fängerstruktur über kontrollierte enzymatische Konjugation eines separaten Adpaterliganden oder einer integrierten Konjugationsstruktur mit dem Oberflächenprotein an der Zelloberfläche exponiert wird, wobei die Fängerstruktur das oder die für die Selektion zu verwendenden Epitope präsentiert. Im Regelfall ist es wünschenswert, die enzymatische Konjugation mit der Ligationspeptidsequenz von der Kopplung der molekularen Fängerstruktur zu trennen, um bezüglich der Auswahl der molekularen Fängerstruktur flexibel zu bleiben. Dafür hat es sich bewährt, die Ligationspeptidsequenz für eine enzymatische Konjugation mit Biotin zu optimieren und die molekulare Fängerstruktur kovalent an ein Andockmolekül, wie beispielsweise einen der Biotinliganden Avidin, Streptavidin oder Neutravidin zu binden und zwar abseits der für das freigesetzte Biomolekül spezifischen Bindungsstelle.

Für das angestrebte Selektionsverfahren ist es außerdem von Bedeutung, Zellen bereit zu stellen, bei denen die Fängerstruktur über ihre spezifische Bindungsstelle für das freigesetzte Biomolekül zwischen einem solchen Biomolekül und dem Oberflächenprotein gebunden ist, um das so präsentierte Biomolekül und somit die das Biomolekül produziernde Zelle mittels eines Detektionsmoleküls identifizieren zu können, wobei es auch möglich ist, auf ein separates Detektionsmolekül zu verzichten und beispielsweise eine Isotopenmarkierung über die Vermehrung der Zelle in isotopenhaltigem Medium direkt in das Biomolekül einzubauen.

Weiterhin ist es für die flexible Handhabung des angestrebten Verfahrens vorteilhaft, wenn Zellen zur Verfügung gestellt werden, bei denen ein separater Adapterligand an die Ligationspeptidsequenz gebunden ist, wobei die Bindung bevorzugt kovalent sein sollte. Ist nun an eine solche adapterisierte Zelle eine Fängerstruktur, zumeist nicht-kovalent, gebunden, kann diese Zelle für das angestrebte Verfahren verwendet werden. Dabei muss die Fängerstruktur nicht zwingend über ein zusätzliches Andockmolekül an den Adapterliganden gebunden sein, im Falle von Biotin als Adapterliganden hat es sich jedoch bewährt, eine kovalent an einen als Andockmolekül fungierenden Biotinliganden gebundene molekulare Fängerstruktur zur Kopplung zu verwenden. Da die nicht-kovalente Bindung von Biotin zu beispielsweise Streptavidin zu den stärksten bekannten Bindungen in biologischen Systemen zählt, hat die hohe Affinität zwischen Adapterligand und Andockmolekül keinen negativen Einfluss auf die Spezifität des Selektionsverfahrens. Bei der Verwendung anderer Adapterliganden und Andockmoleküle ist im Falle von nicht-kovalenten Bindungen zwischen diesen beiden Bindungspartnern auf deren ausreichende Affinität zueinander zu achten, notfalls muss auf kovalente Bindung ausgewichen werden, was beispielsweise durch eine Integration von Adapter- und Andockmolekül in einem Molekül erreicht werden kann.

Das Oberflächenprotein sollte, wie bereits erwähnt, bestimmte Eigenschaften aufweisen, um das angestrebte Selektionsverfahren zu ermöglichen. Entscheidend ist dabei, dass das Oberflächenprotein eine extrazellulär exponierbare Ligationspeptidsequenz aufweist, die eine enzymatische Konjugation eines Adapterliganden, wie vorzugsweise Biotin, erlaubt, wobei der Adapterligand, wie oben dargestellt, auch bereits in das Andockmolekül und/oder die molekulare Fängerstruktur integriert sein kann. Als vorteilhaft hat es sich erwiesen, wenn die Ligationspeptidsequenz eine Biotinakzeptorpeptidsequenz aufweist, bevorzugt die Aminosäurenabfolge GLNDIFEAQKIEWHE vom N- zum C-Terminus, oder mit einer solchen übereinstimmt.

Vorteilhaft ist es, wenn das Oberflächenprotein bereits so konstruiert ist, dass es unabhängig von der Anwesenheit anderer Membranproteine in der Zellmembran verankert ist. Dafür ist es von Vorteil, wenn das Oberflächenprotein wenigstens eine hydrophobe Domäne zum Einbau in eine Zellmembran aufweist. Als günstig hat es sich erwiesen, wenn die Ligationspeptidsequenz des Oberflächenproteins bezogen auf dessen Primärstruktur näher am N-Terminus lokalisiert ist als die hydrophobe Domäne. Besonders günstig für die gewünschte Verankerung des Oberflächenproteins in der Zellmembran ist es, wenn die Peptidsequenz für die hydrophobe Domäne eine Transmembrandomäne des EGF-Rezeptors aufweist, wobei EGF für "epidermal growth factor", zu Deutsch "epidermaler Wachstumsfaktor", steht. Vorzugsweise weist die Peptidsequenz für die hydrophobe Domäne des Oberflächenproteins die Aminsäurenabfolge IATGMVGALLLLLVVALGIGLFM vom N- zum C-Terminus auf oder stimmt mit ihr überein.

Es ist zwar denkbar, die Ligationspeptidsequenz primärstrukturell in unmittelbarer Nachbarschaft zu der hydrophoben Domäne anzuordnen, jedoch ist es für den Zweck, die Ligationspeptidsequenz des Oberflächenproteins extrazellulär zu exponieren günstiger, primärstrukturell zwischen der Ligationspeptidsequenz und der hydrophoben Domäne einen Amionsäurelinker zu platzieren, der vorteilhafterweise die extrazelluläre Domäne des reifen humanen EGF-Rezeptors ganz oder teilweise aufweist, vorzugsweise mit der Aminosäurenabfolge GAPATGSSGLEEKKVCQGTSNKLTQLGTFEDHFLSLQRMFNNCEVVLGNLEITYVQRNYDL SFLKTIQEVAGYALIALNTVERIPLENLQIIRGNMYYENSYALAVLSNYDANKTGLKELPMRNL QEILHGAVRFSNNPALCNVESIQWRDIVSSDFLSNMSMDFQNHLGSCQKCDPSCPNGSCW GAGEENCQRLTKIICAQQCSGRCRGKSPSDCCHNQCAAGCTGPRESDCLVCRKFRDEATC KDTCPPLMLYNPTTYQMDVNPEGKYSFGATCVKKCPRNYVVTDHGSCVRACGADSYEME EDGVRKCKKCEGPCRKVCNGIGIGEFKDSLSINATNIKHFKNCTSISGDLHILPVAFRGDSFT HTPPLDPQELDILKTVKEITGFLLIQAWPENRTDLHAFENLEIIRGRTKQHGQFSLAVVSLNIT SLGLRSLKEISDGDVIISGNKNLCYANTINWKKLFGTSGQKTKIISNRGENSCKATGQVCHAL CSPEGCWGPEPRDCVSCRNVSRGRECVDKCNLLEGEPREFVENSECIQCHPECLPQAMNI TCTGRGPDNCIQCAHYIDGPHCVKTCPAGVMGENNTLVWKYADAGHVCHLCHPNCTYGCT GPGLEGCPTNGPKIPS vom N- zum C-Terminus.

Um unabhängig von den für das angestrebte optimierte Selektionsverfahren gewünschten funktionalen Eigenschaften des Oberflächenproteins dessen Anwesenheit an der Zelloberfläche im Vorfeld der Verfahrensdurchführung überprüfen zu können, hat es sich als günstig erwiesen, das Oberflächenprotein zusätzlich mit einem Kontrollepitop auszustatten, das extrazellulär exponierbar ist, wobei es sich als günstig erwiesen hat, dieses Kontrollepitop primärstrukturell näher am N-Terminus zu platzieren als die Ligationspeptidsequenz. Bevorzugt bildet die Peptidsequenz für das Kontrollepitop ein Hämagglutinin-A-Epitop aus, vorzugsweise mit der Aminosäurenabfolge YPYDVPDYA vom N- zum C-Terminus. Nach spezifischer Interaktion mit einem gegen dieses Kontrollepitop gerichteten Antikörper und dessen Markierung kann beispielsweise cytometrisch oder fluoreszenzmikroskopisch bewertet werden, ob das Oberflächenprotein an der Zellmembran präsent ist.

Damit der Nachteil des Stands der Technik, bei dem nicht auszuschließen ist, dass Interaktionen von Antikörpern oder Reagenzien mit der Zelloberfläche Stoffwechselkaskaden und Rückkopplungen im Zellstoffwechsel auslösen, die sich negativ auf die Vitalität der Zelle und/oder deren Fähigkeit zur Vermehrung, Produktion und/oder Freisetzung von gewünschten Biomolekülen sowie Produktion und/oder Lokalisation des Oberflächenproteins auswirken, ist es von Bedeutung für die Optimierung des angestrebten Verfahrens, dass die während und nach der enzymatischen Konjugation des Ligationspeptids stattfindende Interaktion des Ligationspeptids keinen der benannten negativen Effekte auslöst. Besonders sicher ist daher ein Oberflächenprotein mit einer Gesamtstruktur, die frei ist von Aktin-, DNA- oder RNA-bindenden Motiven, von Kernlokalisierungssignalen, von Golgi-Apparat oder ER-Retentionssignalen, von Signalen für den Transport von der Zellmembran zum Golgi-Apparat sowie, primärstrukturell zumindest zwischen hydrophober Domäne und C-Terminus, frei von phosphorylierbaren Tyrosinresten, vorzugsweise mit der Aminosäurenabfolge LEEGSSKLGSSGYPYDVPDYAGAQPARSGGGLNDIFEAQKIEWHEGAPATGSSGLEEKKV CQGTSNKLTQLGTFEDHFLSLQRMFNNCEVVLGNLEITYVQRNYDLSFLKTIQEVAGYALIAL NTVERIPLENLQIIRGNMYYENSYALAVLSNYDANKTGLKELPMRNLQEILHGAVRFSNNPAL CNVESIQWRDIVSSDFLSNMSMDFQNHLGSCQKCDPSCPNGSCWGAGEENCQRLTKIICA QQCSGRCRGKSPSDCCHNQCAAGCTGPRESDCLVCRKFRDEATCKDTCPPLMLYNPTTY QMDVNPEGKYSFGATCVKKCPRNYVVTDHGSCVRACGADSYEMEEDGVRKCKKCEGPC RKVCNGIGIGEFKDSLSINATNIKHFKNCTSISGDLHILPVAFRGDSFTHTPPLDPQELDILKTV KEITGFLLIQAWPENRTDLHAFENLEIIRGRTKQHGQFSLAVVSLNITSLGLRSLKEISDGDVII SGNKNLCYANTINWKKLFGTSGQKTKIISNRGENSCKATGQVCHALCSPEGCWGPEPRDC VSCRNVSRGRECVDKCNLLEGEPREFVENSECIQCHPECLPQAMNITCTGRGPDNCIQCA HYIDGPHCVKTCPAGVMGENNTLVWKYADAGHVCHLCHPNCTYGCTGPGLEGCPTNGPKI PSIATGMVGALLLLLVVALGIGLFMRRRHIVR vom N- zum C-Terminus.

Damit das Oberflächenprotein wenigstens teilweise die Zellmembran überwinden und die gewünschten Strukturen an der Zelloberfläche präsentieren kann, ist es im Regelfall nötig, dass es cytoplasmatisch als Vorstufe translatiert wird, die durch ein Signalpeptid gekennzeichnet ist, wobei das Signalpeptid regelmäßig im Zuge der Translokation abgespalten, also prozessiert wird. Ein für das angestrebte optimierte Selektionsverfahren im Regelfall bereitzustellendes Mittel ist daher eine Nukleinsäure mit einer Abfolge von Basen oder Basenpaaren, die für das Oberflächenprotein mit einem Signalpeptid am N-Terminus codiert, wobei das Signalpeptid die biologische Information zur Initiierung des zellulären Proteintransports in oder durch Biomembranen aufweist. Als vorteilhaft hat es sich erwiesen, wenn das Signalpeptid die Signalsequenz des EGF-Rezeptorproteins aufweist, vorzugsweise die Aminosäurenabfolge MRPSGTAGAALLALLAALCPASRA vom N- zum C-Terminus.

Die Bereitstellung der Nukleinsäure erfolgt sinnvollerweise dergestalt, dass sie entweder ununterbrochen oder durch weitere Basen oder Basenpaare unterbrochen in einen Expressionsvektor integriert ist, wobei der Fachmann zusätzlich für eine effektive Expression förderliche Promotoren auszuwählen weiß.

Der Expressionsvektor wiederum wird für das angestrebte optimierte Selektionsverfahren in eine Zelle eingebracht. Sollen Hybridomzellen, die monoklonale Antikörper produzieren dem angestrebten Verfahren unterzogen werden, so ist es von Vorteil, den Expressionsvektor vor der Fusion von B-Zellen und Myelomzellen zu Hybridomzellen in die Myelomzellen einzubringen und nachfolgend die Fusion erfolgen zu lassen.

Um von Anfang an mit Zellen arbeiten zu können, die erfolgreich mit dem Expressionsvektor für die Nukleinsäure des Oberflächenproteins oder dessen Vorstufe transfiziert werden konnten, ist es von Vorteil, wenn der Expressionsvektor ein Resistenzgen gegen ein Antibiotikum oder ein Zelltoxin aufweist, wobei sich für Resistenzen gegen Aminoglykosidantibiotika, wie beispielsweise die Gentamycine, codierende Resistenzgene als vorteilhaft erwiesen haben ebenso wie für Resistenzen gegen das Zelltoxin Puromycin oder dessen Salze codierende Resistenzgene. So können von Anfang an mit einem Wachstumsmedium, das das entsprechende Antibiotikum oder Zelltoxin aufweist, die Zellen gezüchtet werden, die gegen das Antibiotikum oder das Zelltoxin resistent sind und folglich erfolgreich mit dem Expressionsvektor transfiziert wurden und das Oberflächenprotein an ihrer Oberfläche aufweisen.

Mit den Zellen, die den Expressionsvektor enthalten und das Oberflächenprotein an der Zelloberfläche tragen, ist es möglich, das optimierte Verfahren zur Selektion von Zellen durchzuführen, die Biomoleküle freisetzen. Das Verfahren ermöglicht es, aus einer Vielzahl von Zellen diejenigen zu selektieren, die das Biomolekül mit der besten Affinität zu einem bestimmten Zielmolekül aufweisen und am effizientesten produzieren und freisetzen. Ein solches Verfahren umfasst eine enzymatische Konjugation des Adapterliganden mit der Ligationspeptidsequenz, die mittelbare oder unmittelbare Kopplung der molekularen Fängerstruktur abseits der spezifischen Bindungsstelle und die spezifische Bindung wenigstens eines der freigesetzten Biomoleküle an die spezifische Bindungsstelle.

Es umfasst vorteilhafterweise weiterhin die Markierung des an die Fängerstruktur gebundenen Biomoleküls mittels eines an einen Träger gebundenen, gegen das Biomolekül gerichteten Nachweis-Antikörpers, -Antikörperfragments oder einer sonstigen, hochaffin an das Biomolekül bindenden molekularen Detektionsstruktur. Träger und Detektionsstruktur können auch in einem Molekül integriert sein.

In einer bevorzugten Variante des Verfahrens emittiert der Träger oder die molekulare Detektionsstruktur elektromagnetische Wellen und/oder ist magnetisch oder magnetisierbar und/oder ist immobilisiert. Dadurch ist es möglich, dem Fachmann bekannte Detektionsmethoden wie beispielsweise Durchflusszytometrie, Fluoreszenzmikroskopie oder auch Affinitätschromatographie zum Einsatz zu bringen.

Ein besonderer Vorteil des Verfahrens besteht darin, dass es möglich ist, wenn das freigesetzte Biomolekül ein Antikörper oder Antikörperfragment ist und die Detektionsstruktur, der Nachweis-Antikörper oder das Nachweis-Antikörperfragment gegen die isotyp- oder subklassenspezifische Region des freigesetzten Antikörpers gerichtet ist, den Isotyp und/oder die Subklasse des freigesetzten Produkts zu bestimmen. Dies ist deshalb von Vorteil, weil so eine spezifische, als optimal selektierte Zelle direkt in eine Zellkultur überführt werden kann und zugleich bereits Klarheit über die Subklasse des produzierten Antikörpers bzw. -fragments besteht. Für viele Anwendungen sind bestimmte Subklassen bevorzugt, die nach Stand der Technik erst nach Stabilisierung des Hybridoms und entsprechender Aufreinigung des Antikörpers oder -fragments bestimmt werden können.

Zusammengefasst basiert das hier vorgestellte Verfahren darauf, dass eine spezifische Kopplung einer für das von der Zelle freigesetzte Biomolekül spezifischen Bindungsstelle an eine Fängerstruktur gelingt, die es ermöglicht, das freigesetzte Biomolekül spezifisch an der Zelloberfläche zu binden und anschließend zu detektieren, wobei zusätzlich die Vorteile eines universell einsetzbaren Adaptersystems genutzt werden können, so dass das Verfahren nicht auf Antikörper produzierende Zellen beschränkt ist sondern jegliche Zelle, die ein Biomolekül von Interesse produziert und freisetzt, aufgrund der Affinität des Biomoleküls zu einer gewünschten Zielstruktur und der produzierten und freigesetzten Menge des Biomoleküls selektiert werden kann. Dabei liegt der Unterschied zum Stand der Technik insbesondere darin, dass bereits die Bereitstellung der Fängerstruktur an der Zelloberfläche spezifisch erfolgt und ebenso die Bindung des Biomoleküls an die Fängerstruktur spezifisch ist, weil sie eine für das gesuchte Biomolekül möglichst einzigartige Erkennungsstruktur zum Gegenstand hat. Bezogen auf die Selektion monoklonaler Antikörper nach dem neuen Verfahren ist es von Bedeutung, dass zum einen die Fängerstruktur kontrolliert an der Zelloberfläche präsentiert und der von der Zelle freigesetzte monoklonale Antikörper über seine variable, epitopspezifische Bindungsregion an die molekulare Fängerstruktur gebunden wird und zum anderen eine Bindung des freigesetzten monoklonalen Antikörpers über seinen nicht molekülindividuellen Fc-Teil vermieden wird, die von Anfang an die Spezifität der Selektion herabsetzen würde, ohne dass dieser Mangel durch nachfolgende, spezifische Bindungen an Detektionsstrukturen geheilt werden könnte. Für den Erfolg des neuen Verfahrens ebenfalls bedeutsam ist die Möglichkeit der schonenden Zellbehandlung, die es erlaubt, cytometrisch ausgeeinzelte Zellen direkt in die Zellkultur zu überführen. Aufgrund eines in konjugiertem und unkonjugiertem Zustand stoffwechselneutral gestalteten Oberflächenproteins, wird eine verfahrensunabhängige Zellvermehrung, Produktion und Freisetzung sichergestellt. Schließlich kann das Verfahren aufgrund der verwendeten Adaptertechnik für unterschiedlichste Biomoleküle eingesetzt werden und ist somit nicht auf Antikörper beschränkt.

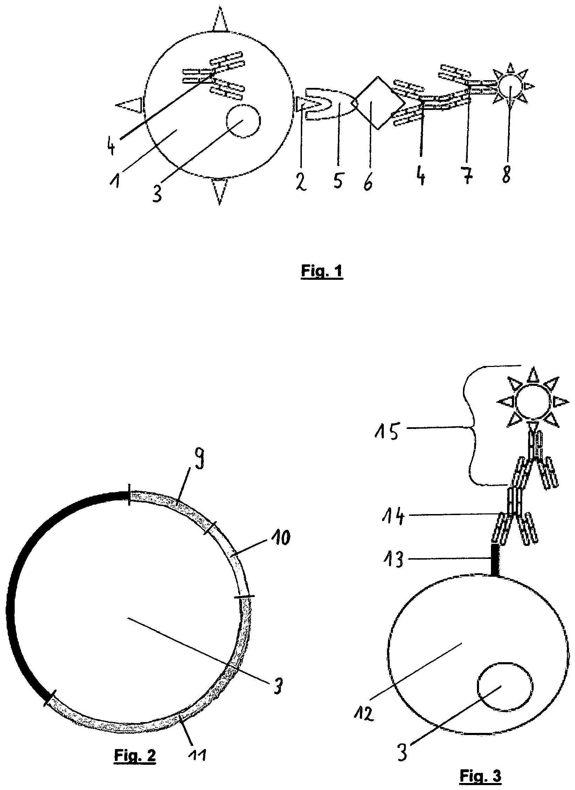

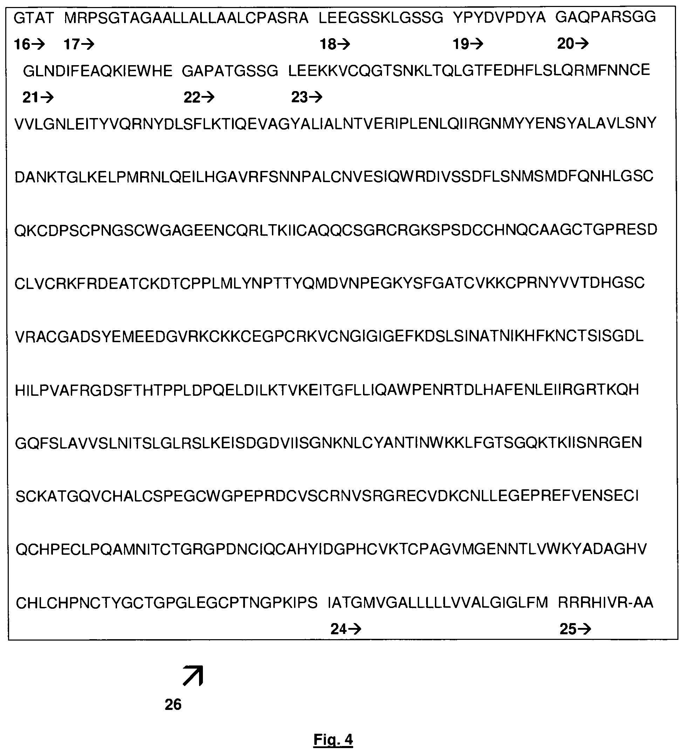

Das erfindungsgemäße Verfahren und die dafür bereit zu stellenden Mittel werden anhand der Zeichnungen und eines Ausführungsbeispiels erläutert, bei dem der in Fig. 2 dargestellte Expressionsvektor für das in Fig. 4 als Primärstruktur dargestellte Oberflächenprotein in Myelomzellen eingebracht wird, die nachfolgend mit B-Lymphozyten, kurz B-Zellen, zu antikörperproduzierenden Hybridomzellen fursioniert werden.

### Leaende:

- 1: Hybridomzelle
- 2: enzymatisch biotinyliertes künstliches Oberflächenprotein
- 3: Expressionsvektor
- 4: monoklonaler Antikörper
- 5: Streptavidin
- 6: streptavidingebundenes Antigen
- 7: Nachweisantiköper
- 8: nachweisantikörpergebundene Fluoreszenzmarkierung
- 9: Signalsequenz
- 10: Hämagglutinin-A-Epitop-Sequenz und Biotinakzeptorpeptid-Sequenz
- 11: Sequenz für den N-terminalen Teil des reifen EGF-Rezeptors
- 12: Myelomzelle
- 13: Hämagglutinin-A-Epitop
- 14: Hämagglutinin-A-Antikörper
- 15: fluoreszenzmarkierter Markierungsantikörper
- 16: Schnittstelle
- 17: Signalqequenz
- 18: Linker
- 19: Hämagglutinin-A-Epitop
- 20: Linker
- 21: Biotinakzeptorpeptid
- 22: Linker
- 23: Extrazelluläre Domäne des EGF-Rezeptors
- 24: Transmembrandomäne des EGF-Rezeptors
- 25: Translationsstopp - Schnittstelle
- 26: Oberflächenprotein einschließlich Signalsequenz

### Beschreibung der Zeichnungen:

**Fig.1** zeigt das dem erfindungsgemäßen Verfahren zugrunde liegende Prinzip. Dabei weist die den monoklonalen Antikörper (4) produzierende Hybridomzelle (1) den Expressionsvektor (3) auf, der ein künstliches Oberflächenprotein einschließlich Signalsequenz (Fig. 4, Ziff. 26) exprimiert und nach Translokation in prozessierter Form an der Zelloberfläche präsentiert, das hier in enzymatisch biotinylierter Form gezeigt ist (2) und an mit dem Antigen (6), gegen das der sezernierte monoklonale Antikörper (4) gerichtet ist, konjugiertes Streptavidin (5) gekoppelt ist, wobei der sezernierte monoklonale Antikörper (4) über eine nachweisantikörpergebundene Fluoreszenzmarkierung (9), die über den Nachweisantiköper (8) an den sezernierten monoklonalen Antikörper (4) gebunden ist, detektierbar ist und mit ihm (4) die ihn (4) freisetzende Hybridomzelle (1).
**Fig. 2** zeigt eine schematische Darstellung der Konstruktion des Inserts für den Expressionsvektor (3) für das Oberflächenproteins (Fig. 4, Ziff. 26) mit der Signalsequenz (9), der Hämagglutinin-A-Epitop-Sequenz und Biotinakzeptor-Sequenz (10) und der Sequenz für den N-terminalen Teil des EGF-Rezeptors (11).
**Fig. 3** zeigt eine schematische Darstellung des Tests auf erfolgreich transfizierte Myelomzellen (12), die den Expressionsvektor (3) aufweisen, mittels des Hämagglutinin-A-Epitops (13), das, integriert an das an der Zelloberfläche exponierte Oberflächenprotein, präsentiert wird und an einen Hämagglutinin-A-Antikörper (14) bindet, der seinerseits an einen fluoreszenzmarkierten Markierungsantikörper (15) bindet und so einer Detektion zugänglich ist.
**Fig. 4** zeigt die Proteinsequenz (26) des 5'3'Frames für das Oberflächenprotein einschließlich der abspaltbaren aminoterminalen Signalsequenz, wobei die einzelnen Proteinregionen voneinander abgesetzt und beziffert sind, obwohl eine durchgehende Polypeptidkette dargestellt ist. Zu erkennen ist am N-Terminus eine Schnittstelle (16), gefolgt von der Signalsequenz (17), gefolgt von einem ersten Linker (18), gefolgt von dem Hämagglutinin-A-Epitop (19), gefolgt von einem zweiten Linker (20), gefolgt von dem Biotinakzeptorpeptid (21), gefolgt von einem dritten Linker (22), gefolgt von der extrazellulären Domäne des EGF-Rezeptors (23), gefolgt von der Transmembrandomäne des EGF-Rezeptors (24) und abschließend eine Translationsstopp - Schnittstelle.

### Konstruktion des Expressionsvektors

Für die Konstruktion des Expressionsvektors werden die DNA-Sequenzen in folgender Reihenfolge vom N- zum C-Terminus kloniert:
Signalpeptid des unreifen humanen EGF-Rezeptors:
   Primer vorwärts 5'ATATAGGTACCGCCACCATGCGACCCTCCG 3'
   Primer rückwärts 5'ATTATAAGCTTAGACGAGCCTTCCTCCAGAGCC 3'
Hämagglutinin-Epitop mit Biotin-Akzeptor-Peptid:
   Primer vorwärts 5'CATGAAAGCTTGGCTCGTCTGGGTATCCATATGATG 3'
   Primer rückwärts 5'CTAATGGTAGCCGGCGCGCCCTCG 3'
Extrazelluläre Domäne und Transmembrandomäne des reifen humanen EGF-Rezeptors:
   Primer vorwärts 5'ATAGAGCTACCGGAAGCAGCGGGCTGGAGGAAAAGA 3'
   Primer rückwärts 5'CATAAGCGGCCGCTTACCGAACGATGTGG 3'

Konstruktion des Plamids pCEP4 mit der Sequenz für das Oberflächenprotein einschließlich der Signalsequenz, Schnittstellen sind unterstrichen, die Kozak-Sequenz fett gedruckt gefolgt vom Start-ATG für die Signalsequenz:

GGTACCGCCACCATGCGACCCTCCGGGACGGCCGGGGCAGCGCTCCTGGCGCTGCT
GGCTGCGCTCTGCCCGGCGAGTCGGGCTCTGGAGGAAGGCTCGTCTAAGCTTGGCTC
GTCTGGGTATCCATATGATGTTCCAGATTATGCTGGGGCCCAGCCGGCCAGATCTGGC
GGCGGCCTGAACGACATCTTCGAGGCCCAGAAGATCGAGTGGCACGAGGGCGCGCCG
GCTACCGGaAGCAGCGGGCTGGAGGAAAAGAAAGTTTGCCAAGGCACGAGTAACAAGC
TCACGCAGTTGGGCacTTTTGAAGATCATTTTCTCAGCCTCCAGAGGATGTTCAATAACT
GTGAGGTGGTCCTTGGGAATTTGGAAATTACCTATGTGCAGAGGAATTATGATCTTTCCT
TCTTAAAGACCATCCAGGAGGTGGCTGGTTATGCCCTCATTGCCCTCAACACAGTGGAG
CGAATTCCTTTGGAAAACCTGCAGATCATCAGAGGAAATATGTACTACGAAAATTCCTAT
GCCTTAGCAGTCTTATCTAACTATGATGCAAATAAAACCGGACTGAAGGAGCTGCCCAT
GAGAAATTTACAGGAAATCCTGCATGGCGCCGTGCGGTTCAGCAACAACCCTGCCCTG
TGCAACGTGGAGAGCATCCAGTGGCGGGACATAGTCAGCAGTGACTTTCTCAGCAACA
TGTCGATGGACTTCCAGAACCACCTGGGCAGCTGCCAAAAGTGTGATCCAAGCTGTCC
CAATGGGAGCTGCTGGGGTGCAGGAGAGGAGAACTGCCAGAGACTGACCAAAATCATC
TGTGCCCAGCAGTGCTCCGGGCGCTGCCGTGGCAAGTCCCCCAGTGACTGCTGCCAC
AACCAGTGTGCTGCAGGCTGCACAGGCCCCCGGGAGAGCGACTGCCTGGTCTGCCGC

AAATTCCGAGACGAAGCCACGTGCAAGGACACCTGCCCCCACTCATGCTCTACAACC
CCACCACGTACCAGATGGATGTGAACCCCGAGGGCAAATACAGCTTTGGTGCCACCTG
CGTGAAGAAGTGTCCCCGTAATTATGTGGTGACAGATCACGGCTCGTGCGTCCGAGCC
TGTGGGGCCGACAGCTATGAGATGGAGGAAGACGGCGTCCGCAAGTGTAAGAAGTGC
GAAGGGCCTTGCCGCAAAGTGTGTAACGGAATAGGTATTGGTGAATTTAAAGACTCACT
CTCCATAAATGCTACGAATATTAAACACTTCAAAAACTGCACCTCCATCAGTGGCGATCT
CCACATCCTGCCGGTGGCATTTAGGGGTGACTCCTTCACACATACTCCTCCTCTGGATC
CACAGGAACTGGATATTCTGAAAACCGTAAAGGAAATCACAGGGTTTTTGCTGATTCAG
GCTTGGCCTGAAAACAGGACGGACCTCCATGCCTTTGAGAACCTAGAAATCATACGCG
GCAGGACCAAGCAACATGGTCAGTTTTCTCTTGCAGTCGTCAGCCTGAACATAACATCC
TTGGGATTACGCTCCCTCAAGGAGATAAGTGATGGAGATGTGATAATTTCAGGAAACAA
AAATTTGTGCTATGCAAATACAATAAACTGGAAAAAACTGTTTGGGACCTCCGGTCAGAA
AACCAAAATTATAAGCAACAGAGGTGAAAACAGCTGCAAGGCCACAGGCCAGGTCTGC
CATGCCTTGTGCTCCCCCGAGGGCTGCTGGGGCCCGGAGCCCAGGGACTGCGTCTCT
TGCCGGAATGTCAGCCGAGGCAGGGAATGCGTGGACAAGTGCAACCTTCTGGAGGGT
GAGCCAAGGGAGTTTGTGGAGAACTCTGAGTGCATACAGTGCCACCCAGAGTGCCTGC
CTCAGGCCATGAACATCACCTGCACAGGACGGGGACCAGACAACTGTATCCAGTGTGC
CCACTACATTGACGGCCCCCACTGCGTCAAGACCTGCCCGGCAGGAGTCATGGGAGAA
AACAACACCCTGGTCTGGAAGTACGCAGACGCCGGCCATGTGTGCCACCTGTGCCATC
CAAACTGCACCTACGGATGCACTGGGCCAGGTCTTGAAGGCTGTCCAACGAATGGGCC
TAAGATCCCGTCCATCGCCACTGGGATGGTGGGGGCCCTCCTCTTGCTGCTGGTGGTG
GCCCTGGGGATCGGCCTCTTCATGCGAAGGCGCCACATCGTTCGGTAAGCGGCCGCT
CGAGGCCGG

Die Klonierung erfolgt über Standard-PCR-Techniken. Das zusammengesetzte Konstrukt wird ebenfalls über PCR in den pPB EF1alpha Vektor kloniert, um eine Transfektion mittels piggyBac-Transposase System zu ermöglichen.

Die Proteinsequenz des 5'3'Frame lautet 
GTATMRPSGTAGAALLALLAALCPASRALEEGSSKLGSSGYPYDVPDYAGAQPARSGGGL
NDIFEAQKIEWHEGAPATGSSGLEEKKVCQGTSNKLTQLGTFEDHFLSLQRMFNNCEVVLG
NLEITYVQRNYDLSFLKTIQEVAGYALIALNTVERIPLENLQIIRGNMYYENSYALAVLSNYDA
NKTGLKELPMRNLQEILHGAVRFSNNPALCNVESIQWRDIVSSDFLSNMSMDFQNHLGSCQ
KCDPSCPNGSCWGAGEENCQRLTKIICAQQCSGRCRGKSPSDCCHNQCAAGCTGPRESD
CLVCRKFRDEATCKDTCPPLMLYNPTTYQMDVNPEGKYSFGATCVKKCPRNYVVTDHGSC
 
VRACGADSYEMEEDGVRKCKKCEGPCRKVCNGIGIGEFKDSLSINATNIKHFKNCTSISGDL
HILPVAFRGDSFTHTPPLDPQELDILKTVKEITGFLLIQAWPENRTDLHAFENLEIIRGRTKQH
GQFSLAVVSLNITSLGLRSLKEISDGDVIISGNKNLCYANTINWKKLFGTSGQKTKIISNRGEN
SCKATGQVCHALCSPEGCWGPEPRDCVSCRNVSRGRECVDKCNLLEGEPREFVENSECI
QCHPECLPQAMNITCTGRGPDNCIQCAHYIDGPHCVKTCPAGVMGENNTLVWKYADAGHV
CHLCHPNCTYGCTGPGLEGCPTNGPKIPSIATGMVGALLLLLVVALGIGLFMRRRHIVR-AA
 und beginnt mit einer Schnittstelle (Aminosäuren 1 bis 4), gefolgt von der Signalsequenz (Aminosäuren 5 bis 28), gefolgt von einem Linker (Aminosäuren 29 bis 40), gefolgt von dem Hämagglutinin-A-Epitop (Aminosäuren 41 bis 49), gefolgt von einem Linker (Aminosäuren 50 bis 58), gefolgt von der Biotinylierungssequenz (Aminosäuren 59 bis 73), gefolgt von einem Linker (Aminosäuren 74 bis 82), gefolgt von der extrazellulären Domäne des EGF-Rezeptorproteins (Aminosäuren 83 bis 703), gefolgt von der hydrophoben Transmembrandomäne des EGF-Rezeptorproteins (Aminosäuren 704 bis 726), gefolgt von einem Translationsstopp und einer Schnittstelle (Aminosäuren 727 bis 735).

### Kontrolle der erfolgreichen Expression und Oberflächenpräsentation des Oberflächenproteins über das Hämagglutinin-Epitop

3x10⁵ erfolgreich transfizierte Myelomzellen werden mittels Imunfluoreszenz auf die Expression und Oberflächenpräsentation des Oberflächenproteins überprüft. Dafür werden die Myelomzellen auf mit Poly-L-Lysin beschichteten Deckgläschen ausplatiert und in Zellkulturmedium, 2 mL DMEM versetzt mit 3 µg Puromycin, bei 37°C und 8% CO₂ angezogen. Danach werden die Myelomzellen mit steriler phosphatgepufferter Salzlösung (nachfolgend: PBS) gewaschen und mit 4%igem Paraformaldehyd für 20 min fixiert. Nach der Fixierung erfolgt ein Waschschritt mit PBS. Die Deckgläschen werden anschließend 20 min bei Raumtemperatur in einer Blocklösung, 1% Rinderserumalbumin in PBS, inkubiert, danach gewaschen und mit einem Maus-anti-Hämagglutinin Antikörper, 1 µg auf 1x10⁶ Zellen, und einem Phycoerythrin-markierten F(ab)₂-Fragment eines Esel-anti-Maus-Antikörpers, 0,5 µg/mL in Blocklösung, enthaltend 1x PBS versetzt mit 5% neonatalen Kälberserum, gefärbt. Anschließend werden die Deckgläschen zweimal mit PBS und zweimal mit destilliertem Wasser gewaschen und mit einem Einbettmedium konserviert. Die Analyse der Färbung erfolgt mittels eines Fluoreszenzmikroskops.

### Gewinnung einer für die Fusion vorgesehenen antikörperproduzierenden B-Zelle

Um antigenspezifische B-Lymphozyten in einer Maus zu induzieren, werden die Mäuse mit dem Antigen der Wahl immunisiert. Dafür wird je nach Art des Antigens eine entsprechende Immunisierungsprozedur erarbeitet. Für Antigene über 25 kDA erfolgt dies standardmässig ohne weitere Kopplungsprozeduren. Antigene unterhalb dieser Größe werden für eine adäquate Immunantwort an ein größeres Trägerprotein, wie beispielsweise Rinderserumalbumin, Ovalbumin oder Napfschneckenhämocyanin gekoppelt. Die Erst-Immunisierung beinhaltet vorzugsweise 100 µg Antigen und wird zusammen mit einem Adjuvanz intraperitoneal verabreicht. Nach 4-8 Wochen erfolgt eine zweite Immunisierung ohne Adjuvanz und 7 Tage später eine Blutabnahme. Das hergestellte Serum wird mittels eines Standard-Immunoassays auf die Antikörper gegen das immunisierte Antigen getestet, wie in Absatz [0040] näher erläutert. Nach positiver Reaktion der Maus werden deren Milzzellen isoliert und gezählt. Parallel werden Zellen der für die Fusion vorgesehenen Zelllinie ebenfalls vorbereitet und in entsprechender Menge für die Fusion eingesetzt.

### Fusionierung zur Hybridomzelle

Milzzellen und Myelomzellen werden bevorzugt im Verhältnis 3:1 für eine Fusion eingesetzt. Dafür werden die Zellen in Fusionspuffer, nämlich 125mM NaCl, 5 mM KCL, 4 mM CaCl₂, 2,5 mM MgCl₂ und 5 mM Tris-HCI pH=7,4, eingestellt und in Fusionspuffer aufgenommen. Die Fusion der Zellen erfolgt über Elektroporation bei einer Gleichspannung von 500-700 V und einer Impulsdauer von 20 µs. Das Zellpellet wird in 200 µL Fusionspuffer resuspendiert und mit 200 µL PEG8000-Lösung gemischt. Anschließend wird die Zelllösung in eine Elektroporationsküvette mit einem Elektrodenabstand von 0,2 cm überführt und die Elektroporation durchgeführt. Danach inkubieren die Zellen weitere 3 min in der Küvette und werden dann in Selektionsmedium, beispielsweise DMEM versetzt mit 20% fötalem Kälberserum, 2 mM Glutamin, 50 µM beta-Mercaptoethanol, 200 µM Hypoxanthin, 12 µM Azaserin, 32 µM 2'Desoxythymidin und 5 µg/mL Puromycin, überführt und bis zur Sortierung kultiviert.

### Enzymatische Biotinylierung der Ligationspeptidsequenz

Die enzymatische Biotinylierung über das transgen vermittelte Akzeptorpeptid erfolgt durch Zugabe von 1 mM ATP, 10 µM Biotin und 0,79 µg pro 1x10⁶ Zellen BirA für 30 min bei Raumtemperatur. Danach können die Zellen mit dem Antigen-Streptavidin-Konjugat beladen werden.

### Kopplung der molekularen Fängerstruktur abseits des Epitops an Streptavidin

Die Kopplung von Antigenen an Streptavidin erfolgt für globuläre Proteine über den homobifunktionellen Quervernetzer Glutaraldehyd. Für Ovalbumin werden 3 mg Protein mit 2 mg Streptavidin und 0,25% Glutaraldehyd in 1 mL 1x PBS, PBS steht für phosphatgepufferte Salzlösung, gemischt und für 2h bei 4°C inkubiert. Anschließend wird der Reaktionsansatz für 1h gegen 5 L 1x PBS dialysiert. Es ist von Vorteil, die Kopplung der Proteine an Streptavidin mithilfe eines Enzymimmunoassays zu überprüfen. Für den Enzymimmunoassay werden 10 µg eines Rinderserumalbumin-Biotin Konjugates in 1 mL 1x PBS verdünnt und zum Beschichten der Mikrotiterplatte verwendet. Die Beschichtung erfolgt über Nacht bei 4°C in einer Feuchtkammer. Nach dem Waschen der Näpfe mit Leitungswasser werden diese anschließend mit einer Blocklösung, enthaltend 1x PBS versetzt mit 5% neonatalen Kälberserum (nachfolgend: NKS), für 1h bei Raumtemperatur (nachfolgend: RT) abgesättigt. Danach erfolgt erneut ein Waschschritt und die Zugabe des Kopplungskonjugates in einer Konzentration von 10 µg/mL verdünnt in der Blocklösung für 1h bei RT. Nach erneutem Waschen wird mittels eines murinen monoklonalen anti-Ovalbumin-Antikörpers, 1 µg/mL verdünnt in Blocklösung, das gekoppelte Antigen gebunden. Die Inkubation erfolgt ebenfalls für 1h bei RT. Die Detektion des anti-Ovalbumin-Antikörpers wird nach dem Waschschritt mittels eines Peroxidase-konjugierten Ziege-anti-Maus Antikörpers in einer Verdünnung von 1:5000 in Blocklösung für 45 min bei RT durchgeführt. Danach erfolgen weitere Waschschritte und die Zugabe einer Substratlösung, nämlich 0,1 M Na₂H₂PO₄, 0,1% Harnstoff/H₂O₂, 1,2 mg/mL Tetramethylbenzidine verdünnt in Ethanol, im Verhältnis 5:4:1. Die Substratreaktion wird dann mit 1 M H₂SO₄ gestoppt und in einem ELISA-Lesegerät bei einer Wellenlänge von 450 nm vermessen.

### Kopplung des Fängerstruktur-Streptavidin-Konjugats an die produzierende Zelle über das biotinvlierte Oberflächenprotein

Für die Kopplung des Fängerstruktur-Streptavidin-Konjugates werden die produzierenden Zellen zuvor für 30 min *in vitro* biotinyliert gemäß Absatz [0039]. Im Anschluss werden 1x10⁶ Zellen für 20 min. durch Zugabe von 33 µg des Antigen-Streptavidin-Konjugats beladen. Anschließend werden die Zellen zentrifugiert und in frischem Zellkulturmedium aufgenommen. Es erfolgte eine 3-4 stündige Inkubation bei 37°C und 8% CO₂, um die Antikörperproduktion der Zellen zu starten.

### Kopplung des Detektionsantikörpers an den gefangenen Antikörper

Die Zellen werden in ein Reaktionsgefäß überführt und mit einer 1%igen Rinderserumalbumin-Lösung, versetzt mit 2 mM Ethylendiamintetraessigsäure, bei 200x g für 8 min. pelletiert. Das Zellpellet wird erneut in Blocklösung gemäß Absatz [0040] resuspendiert und mit Fluoreszeinisothiocyanat-markiertem Ziege-anti-Maus-Antikörper in einer Konzentration von vorzugsweise 1 µg pro 1 x 10⁶ Zellen auf Eis inkubiert.

### Durchflusscvtometrische Identifizierung der optimalen Zelle

Anschließend erfolgt ein erneuter Waschschritt und die Aufnahme des Zellpellets in 300 µL Puffer für einen fluoreszenzaktivierten Zellsortierer, nämlich 0,5% Rinderserumalbumin, 0,01% NaN₃ in PBS. Die positiv markierten Zellen, die den gewünschten Antikörper produzieren, welcher spezifisch sein Antigen auf der Zelle gebunden hat, werden dann durch die Sortierfunktion des Durchflusszytometers heraus sortiert. Die erhaltene Fraktion wird durch Zentrifugation pelletiert werden.

### Überführung der optimalen Zelle in die Zellkultur

Das Zellpellet wird anschließend in Vollmedium, beispielsweise RPMI 1640, 10% fötales Kälberserum, 2 mM Glutamin, 50 µM beta-Mercaptoethanol, aufgenommen und auf 96well Zellkulturplatten augesät. Die Zellkulturplatten enthalten bereits eine aus dem Peritoneum der Maus isolierte Feederzellpopulation, die das Wachstum der frisch fusionierten und sortierten Hybridomzellen unterstützt.

## Patentansprüche

1. Verfahren zur Selektion von Hybridomzellen aus einer Vielzahl von Zellen, wobei die Hybridomzelle (1) ein Oberflächenprotein (2) mit einer extrazellulär exponierten Ligationspeptidsequenz zur enzymatischen Konjugation eines Adapterliganden aufweist, der geeignet ist für die mittelbare oder unmittelbare Kopplung einer wenigstens eine spezifische Bindungsstelle für die freigesetzten Antikörper (4) aufweisenden molekularen Fängerstruktur abseits der spezifischen Bindungsstelle
umfassend
eine enzymatische Konjugation des Adapterliganden Biotin mit der Ligationspeptidsequenz GLNDIFEAQKIEWHE, die mittelbare oder unmittelbare Kopplung der molekularen Fängerstruktur abseits der spezifischen Bindungsstelle und die spezifische Bindung wenigstens eines der freigesetzten Antikörper (4) an die spezifische Bindungsstelle,
wobei die molekulare Fängerstruktur ein Antigen (6) ist, und die spezifische Bindungsstelle ein Epitop ist, und die freigesetzten Antikörper (4) gegen das Epitop gerichtete Antikörper oder Antikörperbestandteile sind und der Antikörper (4) detektierbar ist.

2. Verfahren nach Anspruch 1,
umfassend
die Bindung des Antigens (6) an ein Andockmolekül (5) und die Bindung des Andockmoleküls an den Adapterliganden Biotin, wobei das Andockmolekül (5) zwischen dem Adapterliganden und dem Antigen (6) gebunden ist, und wobei das Andockmolekül (5) Streptavidin, Avidin oder Neutravidin aufweist.

3. Verfahren nach einem der vorstehenden Ansprüche,
umfassend, eine stabile Expression eines für das Oberflächenprotein (2) codierenden Vektors (3) in der Hybridomzelle (1).

4. Verfahren nach Anspruch 1,
umfassend
die Markierung des an dem Antigen (6) gebundenen Antikörpers mittels eines an einen Träger gebundenen, gegen den Antikörper gerichteten Nachweis-Antikörpers, Antikörperfragments oder einer sonstigen, hochaffin an den Antikörper bindenden molekularen Detektionsstruktur.

5. Verfahren nach Anspruch 4,
**dadurch gekennzeichnet,**
**dass** der Träger oder die molekulare Detektionsstruktur elektromagnetische Wellen emittiert und/oder magnetisch oder magnetisierbar ist und/oder immobilisiert ist.

6. Verfahren nach Anspruch 1,
**dadurch gekennzeichnet,**
**dass** ein Nachweis-Antikörper oder das Nachweis-Antikörperfragment gegen die isotyp- oder subklassenspezifische Region des freigesetzten Antikörpers gerichtet ist.

7. Verfahren nach Anspruch 4,
umfassend
die durchflusscytometrische Sortierung der Zellen mittels der Eigenschaften des Trägers oder der molekularen Detektionsstruktur.

8. Verfahren nach Anspruch 1,
**dadurch gekennzeichnet,**
**dass** die Peptidsequenz des Oberflächenproteins (2) eine hydrophobe Domäne aufweist, insbesondere eine Transmembrandomäne des EGF-Rezeptors, vorzugsweise mit der Aminosäurenabfolge IATGMVGALLLLLWALGIGLFM vom N-zum C-Terminus.

9. Verfahren nach Anspruch 1,
**dadurch gekennzeichnet,**
**dass** die Peptidsequenz des Oberflächenproteins (2) ein extrazellulär exponierbares Kontrollepitop aufweist, insbesondere ein Hämagglutinin-A-Epitop, vorzugsweise mit der Aminosäurenabfolge YPYDVPDYA vom N- zum C-Terminus.

10. Oberflächenprotein,
**gekennzeichnet durch**
die Aminosäureabfolge LEEGSSKLGSSG**YPYDVPDYA**GAQPARSGG**GLNDIFEAQKIEWHE**GAPATGSSGL EEKKVCQGTSNKLTQLGTFEDHFLSLQRMFNNCEWLGNLEITYVQRNYDLSFLKTI QEVAGYALIALNTVERIPLENLQIIRGNMYYENSYALAVLSNYDANKTGLKELPMRNL QEILHGAVRFSNNPALCNVESIQWRDIVSSDFLSNMSMDFQNHLGSCQKCDPSCPN GSCWGAGEENCQRLTKIICAQQCSGRCRGKSPSDCCHNQCAAGCTGPRESDCLV CRKFRDEATCKDTCPPLMLYNPTTYQMDVNPEGKYSFGATCVKKCPRNYVVTDHG SCVRACGADSYEMEEDGVRKCKKCEGPCRKVCNGIGIGEFKDSLSINATNIKHFKN CTSISGDLHILPVAFRGDSFTHTPPLDPQELDILKTVKEITGFLLIQAWPENRTDLHAF ENLEIIRGRTKQHGQFSLAWSLNITSLGLRSLKEISDGDVIISGNKNLCYANTINWKKL FGTSGQKTKIISNRGENSCKATGQVCHALCSPEGCWGPEPRDCVSCRNVSRGREC VDKCNLLEGEPREFVENSECIQCHPECLPQAMNITCTGRGPDNCIQCAHYIDGPHC VKTCPAGVMGENNTLVWKYADAGHVCHLCHPNCTYGCTGPGLEGCPTNGPKIPSI **ATGMVGALLLLLWALGIGLFM**RRRHIVR vom N- zum C-Terminus.

## Claims

1. A method for selecting hybridoma cells from a plurality of cells,
the hybridoma cell (1) comprising a surface protein (2) having an extracellularly exposed ligation peptide sequence for enzymatic conjugation of an adapter ligand that is suitable for the direct or indirect coupling of a molecular catcher structure comprising at least one specific binding site for the released antibodies (4) at a distance from the specific binding site,
comprising
an enzymatic conjugation of the adapter ligand biotin with the ligation peptide sequence GLNDIFEAQKIEWHE, the direct or indirect coupling of the molecular catcher structure at a distance from the specific binding site, and the specific binding of at least one of the released antibodies (4) to the specific binding site, wherein the molecular catcher structure is an antigen (5), the specific binding site is an epitope, and the released antibodies (4) are antibodies or antibody constitutes directed against the epitope, and the antibody (4) is detectable.

2. The method according to claim 1,
comprising
the binding of the antigen (6) to a docking molecule (5), and the binding of the docking molecule to the adapter ligand biotin, wherein the docking molecule (5) is bound between the adapter ligand and the antigen (6), and wherein the docking molecule (6) comprises streptavidin, avidin or neutravidin.

3. The method according to any one of the preceding claims,
comprising a stable expression of a vector (3) coding for the surface protein (2) in the hybridoma cell (1).

4. The method according to claim 1,
comprising
the labelling of the antibody bound to the antigen (6) by means of a detection antibody bound to a carrier and directed against the antibody, antibody fragment or another molecular detection structure binding with high affinity to the antibody.

5. The method according to claim 4,
**characterised in that**
the carrier or the molecular detection structure emits electromagnetic waves and/or is magnetic or magnetisable and/or immobilised.

6. The method according to claim 1,
**characterised in that**
a detection antibody or the detection antibody fragment is directed against the isotype- or subclass-specific region of the released antibody.

7. The method according to claim 4,
comprising
the sorting of the cells by flow-through cytometry by means of the properties of the carrier or the molecular detection structure.

8. The method according to claim 1,
**characterised in that**
the peptide sequence of the surface protein (2) comprises a hydrophobic domain, in particular a transmembrane domain of the EGF receptor, preferably with the amino acid sequence IATGMVGALLLLLWALGIGLFM from the N- to the C-terminus.

9. The method according to claim 1,
**characterised in that**
the peptide sequence of the surface protein (2) comprises an extracellularly exposable control epitope, in particular a haemagglutinin-A epitope, preferably with the amino acid sequence YPYDVPDYA from the N- to the C-terminus.

10. A surface protein,
**characterised by**
the amino acid sequence LEEGSSKLGSSG**YPYDVPDYA**GAQPARSGG**GLNDIFEAQKIEWHE**GAPATGSSGL EEKKVCQGTSNKLTQLGTFEDHFLSLQRMFNNCEWLGNLEITYVQRNYDLSFLKTI QEVAGYALIALNTVERIPLENLQIIRGNMYYENSYALAVLSNYDANKTGLKELPMRNL QEILHGAVRFSNNPALCNVESIQWRDIVSSDFLSNMSMDFQNHLGSCQKCDPSCPN GSCWGAGEENCQRLTKIICAQQCSGRCRGKSPSDCCHNQCAAGCTGPRESDCLV CRKFRDEATCKDTCPPLMLYNPTTYQMDVNPEGKYSFGATCVKKCPRNYVVTDHG SCVRACGADSYEMEEDGVRKCKKCEGPCRKVCNGIGIGEFKDSLSINATNIKHFKN CTSISGDLHILPVAFRGDSFTHTPPLDPQELDILKTVKEITGFLLIQAWPENRTDLHAF ENLEIIRGRTKQHGQFSLAVVSLNITSLGLRSLKEISDGDVIISGNKNLCYANTINWKKL FGTSGQKTKIISNRGENSCKATGQVCHALCSPEGCWGPEPRDCVSCRNVSRGREC VDKCNLLEGEPREFVENSECIQCHPECLPQAMNITCTGRGPDNCIQCAHYIDGPHC VKTCPAGVMGENNTLVWKYADAGHVCHLCHPNCTYGCTGPGLEGCPTNGPKIPSI **ATGMVGALLLLLVVALGIGLFM**RRRHIVR from the N- to the C-terminus.

## Revendications

1. Procédé de sélection de cellules d'hybridome à partir d'une multiplicité de cellules, dans lequel la cellule d'hybridome (1) présente une protéine de surface (2) avec une séquence de peptides de ligation à exposition extracellulaire pour la conjugaison enzymatique d'un ligand d'adaptateur, qui est approprié pour le couplage direct ou indirect d'au moins un point de liaison spécifique pour les structures de capture moléculaires présentant les anticorps libérés (4) en aval du point de liaison spécifique
comprenant
une conjugaison enzymatique des ligands d'adaptateur de biotine avec la séquence de peptides de ligation GLNDIFEAQKIEWHE, le couplage direct ou indirect de la structure de capture moléculaire en aval du point de liaison spécifique et la liaison spécifique d'au moins un des anticorps libérés (4) au point de liaison spécifique, dans lequel la structure de capture moléculaire est un antigène (6), et le point de liaison spécifique est un épitope, et les anticorps libérés (4) sont des anticorps ou constituants d'anticorps orientés contre l'épitope et l'anticorps (4) peut être détecté.

2. Procédé selon la revendication 1,
comprenant
la liaison de l'antigène (6) à une molécule d'ancrage (5) et la liaison de la molécule d'ancrage au ligand d'adaptateur de biotine, où la molécule d'ancrage (5) est reliée entre le ligand d'adaptateur et l'antigène (6), et où la molécule d'ancrage (5) présente de la streptavidine, de l'avidine ou de la neutravidine.

3. Procédé selon l'une des revendications précédentes,
comprenant, une expression stable d'un vecteur (3) dans la cellule d'hybridome (1) codant pour la protéine de surface (2).

4. Procédé selon la revendication 1,
comprenant
le marquage de l'anticorps lié à l'antigène (6) au moyen d'un anticorps de détection orienté contre l'anticorps, relié à un support, d'un fragment d'anticorps ou d'un similaire, d'une structure de détection moléculaire se liant avec une affinité élevée à l'anticorps.

5. Procédé selon la revendication 4,
**caractérisé en ce,**
**que** le support ou la structure de détection moléculaire émet des ondes électromagnétiques, et/ou est magnétique ou magnétisable, et/ou est immobilisé.

6. Procédé selon la revendication 1,
**caractérisé en ce,**
**qu'**un anticorps de détection ou le fragment d'un anticorps de détection est orienté contre la région de l'anticorps libéré spécifique à l'isotype ou à la sous classe.

7. Procédé selon la revendication 4,
comprenant
le classement des cellules par cytométrie en flux au moyen des propriétés du support ou de la structure de détection moléculaire.

8. Procédé selon la revendication 1,
**caractérisé en ce,**
**que** la séquence de peptides de la protéine de surface (2) présente des domaines hydrophobes, notamment, des domaines transmembranaires d'un récepteur de l'EGF, de préférence, avec la suite d'acides aminés IATGMVGALLLLLWALGIGLFM à partir de l'extrémité N-terminale à l'extrémité C-terminale.

9. Procédé selon la revendication 1,
**caractérisé en ce,**
**que** la séquence peptidique de la protéine de surface (2) présente un épitope de contrôle à exposition extracellulaire, notamment un épitope d'hémagglutinine-A, de préférence avec la suite d'acides aminés YPYDVPDYA à partir de l'extrémité N-terminale à l'extrémité C-terminale.

10. Protéine de surface,
**caractérisée par**
la suite d'acides aminés LEEGSSKLGSSG**YPYDVPDYA**GAQPARSGG**GLNDIFEAQKIEWHE**GAPATGSSGL EEKKVCQGTSNKLTQLGTFEDHFLSLQRMFNNCEVVLGNLEITYVQRNYDLSFLKTI QEVAGYALIALNTVERIPLENLQIIRGNMYYENSYALAVLSNYDANKTGLKELPMRNL QEILHGAVRFSNNPALCNVESIQWRDIVSSDFLSNMSMDFQNHLGSCQKCDPSCPN GSCWGAGEENCQRLTKIICAQQCSGRCRGKSPSDCCHNQCAAGCTGPRESDCLV CRKFRDEATCKDTCPPLMLYNPTTYQMDVNPEGKYSFGATCVKKCPRNYVVTDHG SCVRACGADSYEMEEDGVRKCKKCEGPCRKVCNGIGIGEFKDSLSINATNIKHFKN CTSISGDLHILPVAFRGDSFTHTPPLDPQELDILKTVKEITGFLLIQAWPENRTDLHAF ENLEIIRGRTKQHGQFSLAVVSLNITSLGLRSLKEISDGDVIISGNKNLCYANTINWKKL FGTSGQKTKIISNRGENSCKATGQVCHALCSPEGCWGPEPRDCVSCRNVSRGREC VDKCNLLEGEPREFVENSECIQCHPECLPQAMNITCTGRGPDNCIQCAHYIDGPHC VKTCPAGVMGENNTLVWKYADAGHVCHLCHPNCTYGCTGPGLEGCPTNGPKIPSI **ATGMVGALLLLLVVALGIGLFM**RRRHIVR à partir de l'extrémité N-terminale à l'extrémité C-terminale.
